# EUROPEAN PATENT APPLICATION

(11) **EP 4 190 244 A1**
(43) Date of publication of application: **07.06.2023**
(21) Application number: 22154677.3
(22) Date of filing: 02.02.2022
(51) Int. Cl.: A61B 8/04, A61B 8/08

(54) **DETERMINING A PRESSURE-RELATED VALUE OF A MITRAL VALVE OF A HEART**

(30) Priority: 01.12.2021 US 202163284782 P
(71) Applicant: Koninklijke Philips N.V., 5656 AG Eindhoven (NL)
(72) Inventor: WEBER, Frank Michael, Eindhoven (NL); LAGHI, Andrea, Eindhoven (NL); WILD, Sebastian, Eindhoven (NL)
(74) Representative: Philips Intellectual Property & Standards

(57) **Abstract**

The present invention relates to assessing the state of a mitral valve. In order to provide a facilitated way of assessing a pressure-related condition of a mitral valve, a device (10) for determining a pressure-related value of a mitral valve of a heart is provided that comprises a data input (12), a data processor (14) and an output interface (16). The data input is configured to provide a first ultrasound image (20) of a heart comprising at least a proximal part of a left atrial appendage of the heart in a first state; and to provide a second ultrasound image (22) of a heart comprising at least the proximal part of the left atrial appendage of the heart in a second state. The first state relates to a first pressure state of the left atrium of the heart and the second state relates to a second pressure state of the left atrium of the heart which second pressure state is different than the first pressure state. The data processor is configured to segment the first ultrasound image generating a first segmentation of at least a part of the left atrial appendage; and to segment the second ultrasound image generating a second segmentation of at least a part of the left atrial appendage; to determine, from the first segmentation, at least one first anatomical measurement relating to the left atrial appendage; and to determine, from the second segmentation, at least one second anatomical measurement relating to the left atrial appendage; and to derive at least one relation of the at least one first anatomical measurement and the at least one second anatomical measurement. The output interface is configured to provide the derived at least one relation as an indicator for a pressure related parameter of the mitral valve.

## Description

### FIELD OF THE INVENTION

The present invention relates to assessing the state of a mitral valve. The present invention relates in particular to a device for determining a pressure-related value of a mitral valve of a heart, to a system for determining a pressure-related value of a mitral valve of a heart and to a method for determining a pressure-related value of a mitral valve of a heart.

### BACKGROUND OF THE INVENTION

Mitral regurgitation is a prevalent heart condition. Because the mitral valve does not close properly, there may be an unphysiological blood flow back to the atria during ventricular contraction. This regurgitant blood flow may lead to an additional, transient pressure increase in the atria during ventricular contraction. It goes on top of the physiological pressure increase in the atria during that phase. For assessing the heart condition, atrial pressure can be measured invasively using catheters or pressure wires. However, the invasive application may make measurements difficult. Alternatively, the pressure can be derived from complex simulations, which need both anatomical input data and data from external blood pressure measurements. It has been shown that this may be time-consuming and not readily available in a cathlab.

### SUMMARY OF THE INVENTION

There may thus be a need to provide a facilitated way of assessing a pressure-related condition of a mitral valve.

The object of the present invention is solved by the subject-matter of the independent claims; further embodiments are incorporated in the dependent claims.

It should be noted that the following described aspects of the invention apply for the device for determining a pressure-related value of a mitral valve of a heart, for the system for determining a pressure-related value of a mitral valve of a heart and also for the method for determining a pressure-related value of a mitral valve of a heart.

According to the present invention, a device for determining a pressure-related value of a mitral valve of a heart is provided. The device comprises a data input, a data processor and an output interface. The data input is configured to provide a first ultrasound image of a heart comprising at least a proximal part of a left atrial appendage (LAA) of the heart in a first state. The data input is also configured to provide a second ultrasound image of a heart comprising at least the proximal part of the left atrial appendage of the heart in a second state. The first state relates to a first pressure state of the left atrium of the heart, and the second state relates to a second pressure state of the left atrium of the heart which second pressure state is different than the first pressure state. The data processor is configured to segment the first ultrasound image generating a first segmentation of at least a part of the left atrial appendage. The data processor is further configured to segment the second ultrasound image generating a second segmentation of at least a part of the left atrial appendage. The data processor is also configured to determine, from the first segmentation, at least one first anatomical measurement relating to the left atrial appendage. The data processor is furthermore configured to determine, from the second segmentation, at least one second anatomical measurement relating to the left atrial appendage. The data processor is furthermore also configured to derive at least one relation of the at least one first anatomical measurement and the at least one second anatomical measurement. The output interface is configured to provide the derived at least one relation as an indicator for a pressure related parameter of the mitral valve.

As an effect, information concerning a pressure-related condition of a mitral valve is provided based on image data, which means less complicated and facilitated data acquisition and processing for achieving valuable information.

According to an example, the at least one relation comprises a relative difference between the at least one first anatomical measurement and the at least one second anatomical measurement. In an option, the derived at least one relation is provided as an indicator for mitral regurgitation.

According to an example, in a first option, the first pressure state and the second pressure state relate to a first point and a second point on one pressure curve of a cardiac cycle, wherein the second point is different from the first point. In a second option, alternatively or in addition, the first pressure state and the second pressure state relate to a pressure state at a first stage of a cardiac treatment and a pressure state at a second stage of the cardiac treatment, the second stage being different in relation to the proceeding of the cardiac treatment than the first stage. As an option, the first pressure state and the second pressure state relate to the same part of a respective cardiac cycle.

According to an example, for the first option, the first pressure state relates to a first point on an atrial pressure curve and the second pressure state relates to a second point on the atrial pressure curve. The first point is a first peak of an a-wave part of the atrial pressure curve and the second point is a second peak of a v-wave part of the atrial pressure curve. Further, the at least one relation of the at least one first anatomical measurement and the at least one second anatomical measurement comprise a determination of a relative difference between the first and the second peak. As an option, the determination of a relative difference between the first and the second peak is provided in relation to an amplitude of the atrial pressure curve.

According to an example, for the second option, the pressure states at the first and second stage, e.g. the pressure states before and after a treatment, relate to a peak of a v-wave part of the atrial pressure curve. The at least one relation of the at least one first anatomical measurement and the at least one second anatomical measurement comprises a determination of the peak of the v-wave part of the atrial pressure curve before the treatment and the peak of the v-wave part of the atrial pressure curve after the treatment. For the determination, a first geometrical measurement is provided for the peak of the v-wave part of the atrial pressure curve at the first stage, e.g. before the treatment, and a second geometrical measurement is provided for the peak of the v-wave part of the atrial pressure curve at the second stage, e.g. after the treatment.

According to an example, the first ultrasound image and the second ultrasound image further comprise a mitral valve area of the heart. As an option, it is provided that the first and second ultrasound images are provided by a mitral valve procedure and are used for the determining of the pressure-related value of a mitral valve of a heart. As another option, alternatively or in addition, it is provided that the first and second ultrasound images are provided for a mitral valve procedure.

In an example, the same two images are provided for a mitral valve procedure and the determination of the indicator for the pressure related parameter of the mitral valve.

According to an example, the device is comprising a display. The display is configured to display the derived at least one relation.

According to the present invention, a system for determining a pressure-related value of a mitral valve of a heart is provided. The system comprises an ultrasound imaging device configured to provide image data of the mitral valve of a heart of a subject. The system also comprises an example of the device according to one of the preceding examples. The ultrasound imaging device provides the first and second ultrasound images of the heart.

According to an example, the ultrasound imaging device comprises a transesophageal imaging probe for acquiring the first and second ultrasound images.

According to the present invention, also a method for determining a pressure-related value of a mitral valve of a heart is provided. The method comprises the following steps:
- Providing a first ultrasound image of a heart comprising at least a proximal part of a left atrial appendage of the heart in a first state; and providing a second ultrasound image of a heart comprising at least the proximal part of the left atrial appendage of the heart in a second state. The first state relates to a first pressure state of the left atrium of the heart and the second state relates to a second pressure state of the left atrium of the heart which second pressure state is different than the first pressure state.
- Segmenting the first ultrasound image generating a first segmentation of at least a part of the left atrial appendage; and segmenting the second ultrasound image generating a second segmentation of at least a part of the left atrial appendage.
- Determining, from the first segmentation, at least one first anatomical measurement relating to the left atrial appendage; and determining, from the second segmentation, at least one second anatomical measurement relating to the left atrial appendage.
- Deriving at least one relation of the at least one first anatomical measurement and the at least one second anatomical measurement.
- Providing the derived at least one relation as an indicator for a pressure related parameter of the mitral valve.

According to an aspect, image data, such as ultrasound images, of the left atrial appendage of the heart is provided for two different pressure states in order to derive knowledge about the prevailing conditions of the mitral valve of a heart. It is provided to derive a pressure-related parameter of the mitral valve of the heart without actually measuring the pressure. The images show anatomical details of at least the proximal part of the left atrial appendage. By providing images that relate to two different, predetermined pressure states, a relation of the two states can be formed that allows to derive conclusions for the pressure related parameter of the mitral valve, e.g. if and to what extent regurgitant blood flow exists.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for determining a pressure-related value of a mitral valve of a heart.
Fig. 2 schematically shows an example of a system for determining a pressure-related value of a mitral valve of a heart.
Fig. 3 shows basic steps of an example of a method for determining a pressure-related value of a mitral valve of a heart
Fig. 4 shows an example of a representation of a left atrial appendage in a segmentation model with indicated anatomical measurements.
Fig. 5 shows am example of pressure curves for acute mitral regurgitation.
Fig. 6 shows a size curve of a left atrial appendage during a cardiac cycle.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

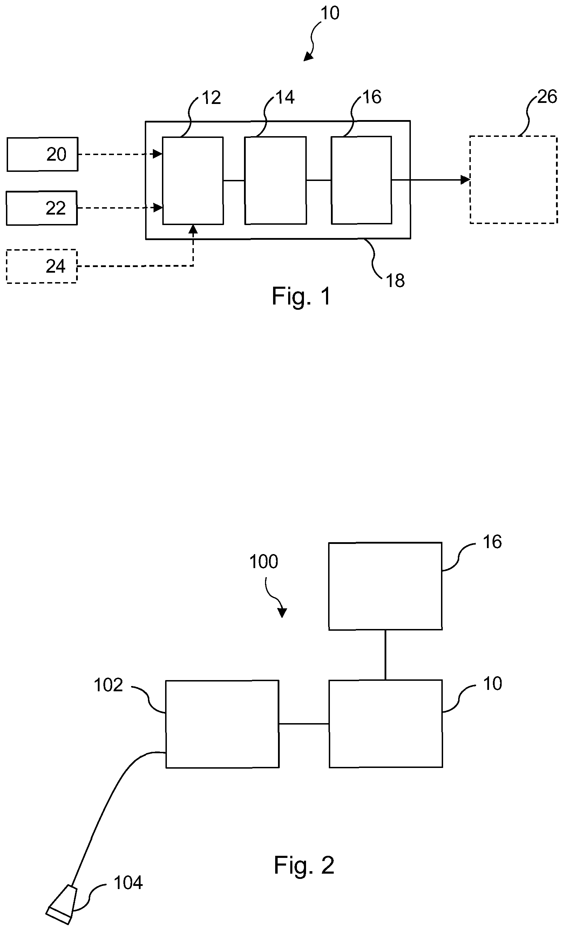

Fig. 1 schematically shows an example of a device 10 for determining a pressure-related value of a mitral valve of a heart. The device 10 comprises a data input 12, a data processor 14 and an output interface 16. A frame 18 indicates an option to provide the data input 12, the data processor 14 and the output interface 16 in an integrated manner, e.g. in a common housing. However, the data input 12, the data processor 14 and the output interface 16 can also be provided as separate components. The data input 12 is configured to provide a first ultrasound image 20 of a heart comprising at least a proximal part of a left atrial appendage of the heart in a first state. The data input 12 is also configured to provide a second ultrasound image 22 of the heart comprising at least the proximal part of the left atrial appendage of the heart in a second state. The first state relates to a first pressure state of the left atrium of the heart and the second state relates to a second pressure state of the left atrium of the heart which second pressure state is different than the first pressure state. The data processor 14 is configured to segment the first ultrasound image generating a first segmentation of at least a part of the left atrial appendage. The data processor 14 is also configured to segment the second ultrasound image generating a second segmentation of at least a part of the left atrial appendage. The data processor 14 is further configured to determine, from the first segmentation, at least one first anatomical measurement relating to the left atrial appendage. The data processor 14 is also configured to determine, from the second segmentation, at least one second anatomical measurement relating to the left atrial appendage. The data processor 14 is still further configured to derive at least one relation of the at least one first anatomical measurement and the at least one second anatomical measurement. The output interface 16 is configured to provide the derived at least one relation as an indicator for a pressure related parameter of the mitral valve.
The data input 12 can also be referred to as data input module. The data processor 14 can also be referred to as data processing module. The output 16 interface can also be referred to as output interface module.

The data input 12 can also be referred to as data supply, as image supply, as image data supply, as input unit or simply as input. In an example, the data input 12 is data-connectable to an imaging source arrangement like an ultrasound imaging arrangement providing the ultrasound image data of the heart which is used for the segmentation. In an example, the (image) data input 12 is data-connectable to a data storage having stored the ultrasound image data. In an example, the (image) data input 12 is data-connectable to a data storage having stored the segmentation of the ultrasound image data.

The data processor 14 can also be referred to as data processing arrangement, as processor unit or as processor. In an example, the data processor 14 is data-connected to the image data input 12 and the output interface 16.

The output interface 16 can also be referred to as output or output unit. In an example, the output interface 16 is data-connectable to a display arrangement or display device. In another example, the output interface 16 is data-connected to a display.

The term "proximal part of the left atrial appendage" refers to the part close to the left atrium.

In an example, the first and/or the second ultrasound image 20, 22 of the heart comprise(s) only the proximal part of the left atrial appendage of the heart. In another example, the first and/or the second ultrasound image 20, 22 of the heart comprise(s) the complete left atrial appendage of the heart.

In an example, not shown in detail, the at least one relation comprises a relative difference between the at least one first anatomical measurement and the at least one second anatomical measurement. The derived at least one relation is provided as an indicator for mitral regurgitation.

In an example, not shown in detail, in a first option, the first pressure state and the second pressure state relate to i) a first point and a second point on one pressure curve of a cardiac cycle, wherein the second point is different from the first point. In a second option, in addition or alternatively, the first pressure state and the second pressure state relate to ii) a pressure state at a first stage of a cardiac treatment and a pressure state at a second stage of the cardiac treatment, the second stage being different in relation to the proceeding of the cardiac treatment than the first stage. As an option, the first pressure state and the second pressure state relate to the same part of a respective cardiac cycle.

As an example, when relating to a pressure at a first and a second point on the curve, an exceeding of the size of the left atrial appendage difference above a predetermined threshold indicates an increased atrial pressure above a predetermined critical value. For example, a relative difference between the a-wave and the v-wave is determined based on the geometrical measurement.

In an example, the first stage of the cardiac treatment relates to a stage before the cardiac treatment and the second stage of the cardiac treatment relates to a stage after the cardiac treatment.

In another example, the first stage of the cardiac treatment relates to a stage before the cardiac treatment and the second stage of the cardiac treatment relates to a stage during the cardiac treatment.

In a further example, the first stage of the cardiac treatment relates to a stage during the cardiac treatment and the second stage of the cardiac treatment relates to a stage after the cardiac treatment.

In a still further example, the first stage of the cardiac treatment relates to a first stage during the cardiac treatment and the second stage of the cardiac treatment relates to a second stage during the cardiac treatment.

As an example, when relating to a pressure before and after the treatment, a decrease in the size of the left atrial appendage indicates a decrease in atrial pressure.

The measurement of the left atrial appendage of the heart provides a surrogate for a pressure measurement.

In an example, only ultrasound images are provided, but no measured pressure data. For example, the images are subject to geometrical measurements, so-to-speak within the image. The geometrical measurements are taken for further processing steps, e.g. calculations, in order to generate or derive an indicator. The indicator for a pressure related parameter, i.e. the pressure is qualitatively determined based on the images.

In an example, not shown in detail, for i), the first pressure state relates to a first point on an atrial pressure curve and the second pressure state relates to a second point on the atrial pressure curve. The first point is a first peak of an a-wave part of the atrial pressure curve and the second point is a second peak of a v-wave part of the atrial pressure curve. The at least one relation of the at least one first anatomical measurement and the at least one second anatomical measurement comprises a determination of a relative difference between the first and the second peak. In an option, the determination of the relative difference between the first and the second peak is provided in relation to an amplitude of the atrial pressure curve.

The a-wave part of the atrial pressure curve relates to an atrial contraction during diastole. Atrial pressure decreases following the a-wave, as the atrium relaxes during systole. The v-wave part of the atrial pressure curve relates to an increase in atrial pressure when the atrium begins to fill with blood during the late systole. The v-wave decreases at the beginning of the diastole, followed by a further a-wave and thus an increase when the contraction starts again.

In an example, a difference is provided between the peak of the v-wave and the baseline of the curve.

In an example, not shown in detail, for ii), the pressure states at the first and second stage relate to a peak of a v-wave part of the atrial pressure curve. The at least one relation of the at least one first anatomical measurement and the at least one second anatomical measurement comprises a determination of the peak of the v-wave part of the atrial pressure curve before the treatment and the peak of the v-wave part of the atrial pressure curve after the treatment. For the determination, a first geometrical measurement is provided for the peak of the v-wave part of the atrial pressure curve before the treatment, and a second geometrical measurement is provided for the peak of the v-wave part of the atrial pressure curve after the treatment.

In an example, it is provided to use a combination of i) and ii), e.g. a single measurement is v-wave peak versus baseline or a-wave, and to provide a comparison of this in different stages of the intervention.

In an example, shown as an option in Fig. 1, the data input 12 is configured to provide an electrocardiogram 24 in order to determine the first point and the second point on the atrial pressure curve. In an example, an electrocardiogram device (not shown) is provided that generates the electrocardiogram 24.

In an example, not shown in detail, the first ultrasound image 20 and the second ultrasound image 22 further comprise a mitral valve area of the heart. As a first option, it is provided that the first and second ultrasound images are provided by a mitral valve procedure and are used for the determining of the pressure-related value of a mitral valve of a heart. As a second option, in addition or alternatively, it is provided that the first and second ultrasound images are provided for a mitral valve procedure.

This provides the effect that an image field of view could be totally unmodified or slightly extended only, if the left atrial appendage is not included in the first place.

In an example, a standard ultrasound mitral valve image is partly enlarged in its field of view to also show the left atrial appendage, or at least the proximal part of the left atrial appendage. In an example, a point of view is used for the transesophageal imaging that is also provided for mitral valve imaging.

The image is used for both purposes. A dual-purpose image is thus provided.

Alternatively, it is provided to acquire image data for the same position and by the same probe, but with different imaging settings.

In an option, a standard mitral valve image is used that is targeted on mitral valve treatment. Further, a stored image is used that is targeted on the left atrial appendage.

The image needed for showing at least the proximal part of the left atrial appendage needs to be a bit broader than the mitral valve image.

As an effect, it is possible to use and gather information from image data that is available anyhow in the context of mitral valve or other cardiac interventions. It is made use of the vicinity of the left atrial appendage to the targeted structure of a mitral valve procedure.

In an example, extra images are avoided. Further, also extra probes are avoided.

In an example, not shown in detail, the data processor 14 is configured to provide the segmenting as at least one of the group of model-based segmentation, deep learning segmentation and manual segmentation based on user input via a user interface.

In an example, the manual segmentation is applied when the deriving of the pressure-related feature is not provided in realtime or live modus.

The deep learning segmentation approach is focusing on so-called anatomical intelligence where, based on training, a convolutional neural network provides the segmentation, for example on the fly, i.e. in realtime.

In an example, not shown in detail, the data processor 14 is configured to segment a mitral valve image. As an option, the data processor 14 is configured to register the segmentation with a model. The data processor 14 is configured to estimate a location of the left atrial appendage in the mitral valve image based on a left atrial appendage location indication of the model.

In an example, a model based segmentation is provided and a registration to the model is not required, as the segmentation is a model.

The location indication by the model is thus used for estimating where the left atrial appendage can be expected. In an example, this information is used to adjust the field of view to include the region in the image that is needed.

As an effect, a left atrial appendage geometry analysis is provided for estimating atrial pressure.

The left atrial appendage geometry analysis is provided by a pressure less determination of a pressure related value.

In an example, shown as an option in Fig. 1, a display 26 is provided that is configured to display the derived at least one relation.

Fig. 2 schematically shows an example of a system 100 for determining a pressure-related value of a mitral valve of a heart. The system 100 comprises an ultrasound imaging device 102 configured to provide image data of the mitral valve of a heart of a subject. The system 100 further comprises an example of the device 10 according to one of the preceding examples. The ultrasound imaging device 102 provides the first and second ultrasound images 20, 22 of the heart. For example, the ultrasound imaging device 102 comprises an ultrasound transducer 104.

In an example, the ultrasound imaging device 102 comprises a transesophageal imaging probe for acquiring the first and second ultrasound images. For example, the ultrasound transducer 104 is provided as the transesophageal imaging probe. The transesophageal imaging probe is also referred to as TEE probe.

In an example, the so-called "v-wave" results from progressive filling and mitral regurgitation may lead to a higher v-wave. Therefore, in an example, the v-wave is provided as a clinical parameter to estimate the severity of mitral regurgitation and as clinical parameter to estimate improvements due to therapies.

In an example, the effect of pressure on left atrial appendage size varies between subjects, depending e.g. on atrial stiffness/compliance. For the same subject, however, it is provided to assess relative changes.

In an option, the segmentation unit detects if the left atrial appendage is properly inside the ultrasound field of view. This may, for example, be achieved as follows for a model-based segmentation approach: It is known which vertices/triangles of the mesh belong to the left atrial appendage (or are needed for the respective measurements). It can be determined which fraction p of those vertices is inside the FOV (or inside a certain safety margin from the FOV border). Ifp falls below a certain threshold (e.g. 80%), a warning can be shown on the display.

According to an example, overall baseline loading conditions may influence the size changes to the regurgitant flow. Such effects may be inferred from left atrial appendage diameter/geometry at other points in the cardiac cycle. In an example, when comparing pre/post-procedure v-wave heights, the minimal size (before the v-wave) is used as indicator for baseline pressure. Also, in a further example, the height of an a-wave during atrial contraction is provided as an indicator for different loading conditions.

It is noted further that changes in overall loading conditions might be inferred from left atrial appendage diameter at other points in cardiac cycle, e.g. height of a-wave during atrial contraction.

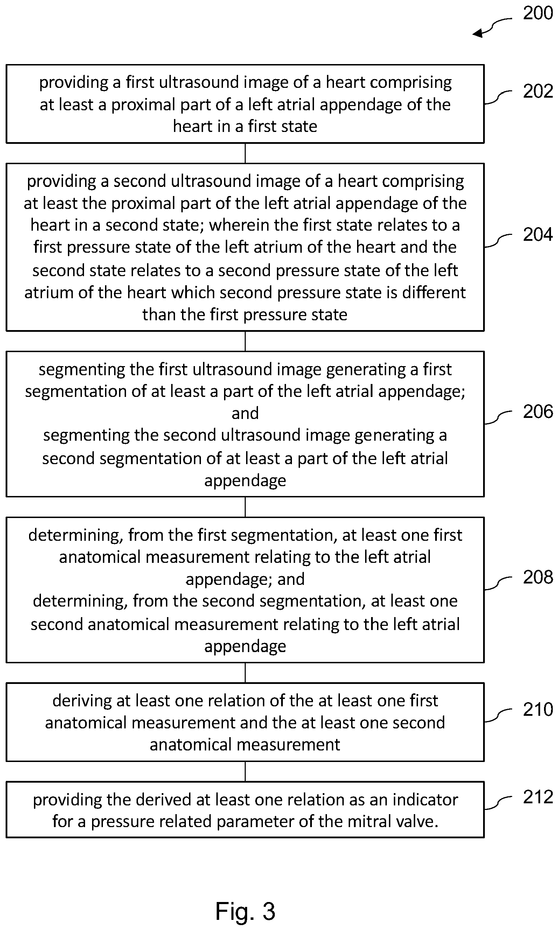

Fig. 3 shows basic steps of an example of a method 200 for determining a pressure-related value of a mitral valve of a heart. The method 200 comprises the following steps: In a first step 202, a first ultrasound image of a heart is provided comprising at least a proximal part of a left atrial appendage of the heart in a first state. In a second step 204, a second ultrasound image of the heart is provided comprising at least the proximal part of the left atrial appendage of the heart in a second state. The first state relates to a first pressure state of the left atrium of the heart and the second state relates to a second pressure state of the left atrium of the heart which second pressure state is different than the first pressure state. In a third step 206, the first ultrasound image is segmented generating a first segmentation of at least a part of the left atrial appendage. Further, also the second ultrasound image is segmented generating a second segmentation of at least a part of the left atrial appendage. In a fourth step 208, from the first segmentation, at least one first anatomical measurement is determined relating to the left atrial appendage; and, from the second segmentation, at least one second anatomical measurement is determined relating to the left atrial appendage. In a fifth step 210, at least one relation of the at least one first anatomical measurement and the at least one second anatomical measurement is derived. In a sixth step 212, the derived at least one relation is provided as an indicator for a pressure related parameter of the mitral valve.

The first step 202 and the second step 204 can be provided as a common step or as two sub-steps of a common step. The fourth step 208 with its two segmentations can also be provided as two segmenting sub-steps.

In an example of the method 200, the at least one relation comprises a relative difference between the at least one first anatomical measurement and the at least one second anatomical measurement.

In an option, the derived at least one relation is provided as an indicator for mitral regurgitation.

In an example of the method 200, the first pressure state and the second pressure state relate to a first point and a second point on one pressure curve. The second point is different from the first point.

In an example of the method 200, the first pressure state and the second pressure state relate to a pressure state at a first stage of a cardiac treatment and a pressure state at a second stage of the cardiac treatment. The second stage is different in relation to the proceeding of the cardiac treatment than the first stage. In an option, the first pressure state and the second pressure state relate to the same part of the cardiac cycle.

In an example of the method 200, the first stage of the cardiac treatment relates to a stage before the cardiac treatment and the second stage of the cardiac treatment relates to a stage after the cardiac treatment. In another example of the method 200, the first stage of the cardiac treatment relates to a stage before the cardiac treatment and the second stage of the cardiac treatment relates to a stage during the cardiac treatment. In a further example of the method 200, the first stage of the cardiac treatment relates to a stage during the cardiac treatment and the second stage of the cardiac treatment relates to a stage after the cardiac treatment. In a still further example of the method 200, the first stage of the cardiac treatment relates to a first stage during the cardiac treatment and the second stage of the cardiac treatment relates to a second stage during the cardiac treatment.

In an example of the method 200, for i), the first pressure state relates to a first point on an atrial pressure curve and the second pressure state relates to a second point on the atrial pressure curve. The first point is a first peak of an a-wave part of the atrial pressure curve and the second point is a second peak of a v-wave part of the atrial pressure curve. Further, the at least one relation of the at least one first anatomical measurement and the at least one second anatomical measurement comprises a determination of a relative difference between the first and the second peak. This can optionally be provided in relation to an amplitude of the atrial pressure curve.

In an example of the method 200, an electrocardiogram is provided in order to determine the first point and the second point on the atrial pressure curve.

In an example, an electrocardiogram (ECG) provides a rough location of the point, but the actual point or location on the curve, e.g. a peak, may be slightly displaced from that point, e.g. a bit off the point. The ECG hence provides a search region over which most or even all anatomical measurements are taken into account, and from those, as an example, the maximum one is selected. Within the region, it is also possible to provide extended calculations, like smoothing the curve (by averaging between neighbor values) and then determining the peak, or fitting a curve (like a Gaussian) to the anatomical measurements and determine the peak height from it. In an example of the method 200, for ii), the pressure state before and after the treatment relate to a peak of a v-wave part of the atrial pressure curve. The at least one relation of the at least one first anatomical measurement and the at least one second anatomical measurement comprise a determination of the peak of the v-wave part of the atrial pressure curve before the treatment and the peak of the v-wave part of the atrial pressure curve after the treatment. For the determination, a first geometrical measurement is provided for the peak of the v-wave part of the atrial pressure curve before the treatment, and a second geometrical measurement is provided for the peak of the v-wave part of the atrial pressure curve after the treatment.

In an example of the method 200, the first ultrasound image and the second ultrasound image further comprise a mitral valve area of the heart.

In an option, the first and second ultrasound images are provided by i) a mitral valve procedure and are used for the determining of the pressure-related value of a mitral valve of a heart. In another option, in addition or alternatively. In a further option, the first and second ultrasound images are ii) provided for a mitral valve procedure.

In an example of the method 200, the segmenting is provided as at least one of the group of model-based segmentation, deep learning segmentation and manual segmentation.

In an example of the method 200, a mitral valve image is segmented. As an option of the method 200 it is provided to register the mitral valve image segmentation with a model. A location of the left atrial appendage in the mitral valve image is estimated based on a left atrial appendage location indication of the model.

As an effect, the solution allows to quantitatively estimate a surrogate for v-wave height and changes in v-wave height directly from cardiac ultrasound images. It leverages information from the left atrial appendage, which is a mainly passive structure, i.e. with negligible self-contraction, so that suitable geometric parameters describing its spatial extent can serve as an indicator for atrial pressure. Possible examples for such parameters are the diameter and cross-sectional area at various positions in the left atrial appendage, the left atrial appendage volume, or more complex quantities designed (and potentially trained) specifically to capture the changes of the left atrial appendage throughout the cardiac cycle which are most relevant for tracking the v-wave.

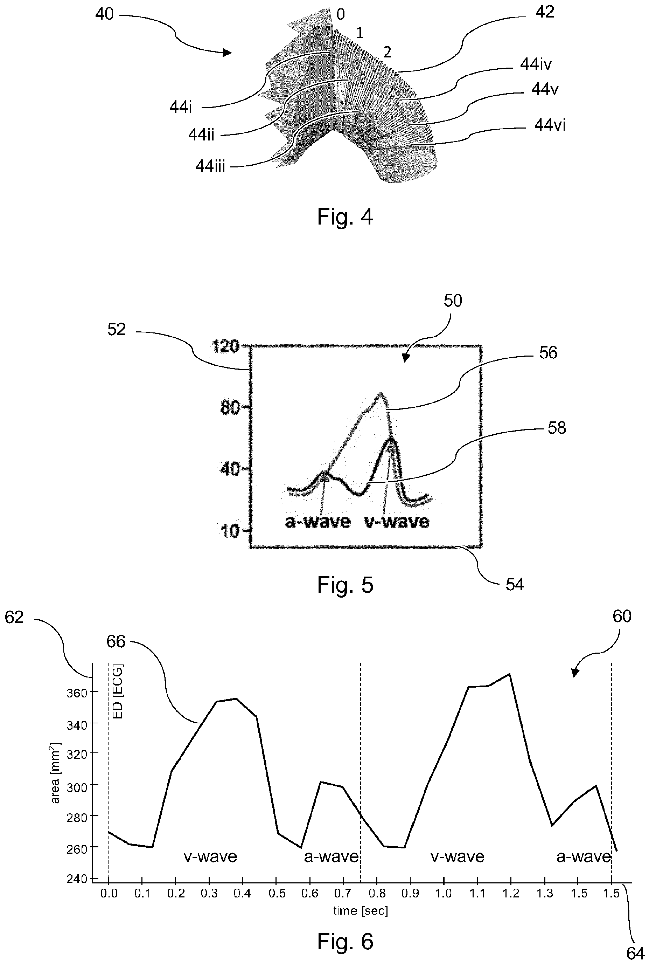

Fig. 4 shows an example of a representation of a left atrial appendage 40 in a segmentation model 42 with indicated anatomical measurements 44. For example, a first anatomical measurement 44i is indicated as a first diameter "0", a second anatomical measurement 44ii is indicated as second diameter "1" and a third anatomical measurement 44iii is indicated as third diameter "2". Further diameters are indicated as anatomical measurements 44iv, 44v and 44vi. The segmentation model 42 may be provided based on a mesh-model. The mesh may carry along further anatomical information, e.g. appropriate locations for anatomical measurements (shown as numbered rings).

Fig. 5 shows examples of pressure curves for acute mitral regurgitation. The pressure is indicated on a vertical axis 52, a horizontal axis 54 indicates the time. A first curve 56 denotes ventricular pressure during contraction (systole). A second curve 58 represents atrial pressure with a pronounced v-wave due to mitral regurgitation. A first peak is indicated with "a-wave" and a second peak is indicated with "v-wave". As can be seen in Fig. 5, the relation of the a-wave and the v-wave can be taken to conclude on the pressure-related status of the mitral valve. If a pronounced v-wave is detected for atrial pressure, the derived indicator provides the respective information that a mitral regurgitation situation of condition is the case. Such pressure-changes are made visible, i.e. detected, by the anatomical images.

Fig. 6 shows a diagram 60 with a size curve of a proximal left atrial appendage during a cardiac cycle. Size changes over the cardiac cycle are visible, and local maxima in size are in agreement with expected local maxima of atrial pressure. A vertical axis 62 indicates area values; a horizontal axis 64 indicates the time. A curve 66 indicates a result for size changes over time. Even though variations occur, respective relations can be detected and a respective indicator for a pressure related parameter of the mitral valve can be generated and provided to the user as useful information.

In an example, an image segmentation method is applied (like model-based segmentation) to measure changes in diameter of the proximal left atrial appendage over the cardiac cycle. An estimate for the height of the v-wave is given by the size changes during systole, e.g. minimal vs. maximal diameter. If one measurement is done before the placement of a device, and another measurement after the placement, the difference may serve as indicator on how much the regurgitation is reduced.

Furthermore, in another example, a common TEE acquisition of both the mitral valve (MV) anatomy and the left atrial appendage is provided, as the left atrial appendage is located close to the mitral valve. This way, in an example, one single field-of-view can capture, for example, both the placement of a mitral clip, and the resulting changes in atrial pressure.

In an example, a system is provided that comprises an acquisition unit. The acquisition unit acquires a series of ultrasound images or receives such a series from another device. The acquisition unit can thus be provided as an ultrasound system or can be connected to an ultrasound system.

In an example, a system is provided that comprises a segmentation unit. The segmentation unit is configured to detect and analyze cardiac structures in images. In an example, model-based segmentation (MBS) is provided, in which a mean model is progressively adapted to the image. For this application, the model-based segmentation model comprises a representation of the proximal left atrial appendage. Because the mesh topology may be fixed, i.e. certain vertices and triangles may be fixed, the mesh may carry along further anatomical information, e.g. appropriate locations for anatomical measurements.

Alternative implementations may be based on approaches like convolutional neural networks or deep learning.

In an example, a system is provided that comprises a measurement unit. The measurement unit is provided to derive relevant anatomical measures from a segmentation result. For example, a mesh can be analyzed at encoded locations to extract geometrical parameters such as local diameter or cross-sectional area of the proximal left atrial appendage. A time course of the extracted left atrial appendage cross-section area from an adapted model may be used. In an example, size changes over the cardiac cycle are visible, and local maxima in size are in agreement with expected local maxima of atrial pressure. In addition, more complex quantities can be derived. For example, size measurements at different locations/depth of the left atrial appendage are combined in an example, to further increase the correspondence with the actual pressure curve.

In another example, machine learning algorithms are provided that are trained using geometrical parameters of the segmentation output and actual pressure measurements how to combine the geometrical measurements from the mesh (e.g. the derived diameters or the set of vertex coordinates itself), with the goal to derive more complex quantities specifically designed to correlate well with the height of the v-wave.

In another example, it is provided to combine the segmentation unit and the measurement unit, e.g. using an end-to-end learning approach that has the image as input and a pressure-related quantity as output.

In an example, a system is provided that comprises an output unit. The output unit is configured to serve as a display on which the derived size curve is displayed, or an interface over which the values can be transferred to a connected system, like a subject monitor, or the display on top of a cathlab table, where they are displayed.

The term "subject" may also be referred to as individual. The "subject" may further also be referred to as patient, although it is noted that this term does not indicate whether any illness or disease is actually present with the subject.

In an example, when in use, the echo operator needs to be instructed to include the left atrial appendage. In another example, the system will also display the measured size curves to allow the physician to double-check the results.

For example, the present solution and its examples are suitable for diagnostic and interventional ultrasound applications, such as the products like Philips EchoNavigator (trademark) or Tomtec Arena (trademark), but also to other products from other manufacturers and vendors.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for determining a pressure-related value of a mitral valve of a heart, the device comprising:
- a data input (12);
- a data processor (14); and
- an output interface (16);
wherein the data input is configured to provide a first ultrasound image (20) of a heart comprising at least a proximal part of a left atrial appendage of the heart in a first state; and to provide a second ultrasound image (22) of a heart comprising at least the proximal part of the left atrial appendage of the heart in a second state; wherein the first state relates to a first pressure state of the left atrium of the heart and the second state relates to a second pressure state of the left atrium of the heart which second pressure state is different than the first pressure state;
wherein the data processor is configured to segment the first ultrasound image generating a first segmentation of at least a part of the left atrial appendage; and to segment the second ultrasound image generating a second segmentation of at least a part of the left atrial appendage; to determine, from the first segmentation, at least one first anatomical measurement relating to the left atrial appendage; and to determine, from the second segmentation, at least one second anatomical measurement relating to the left atrial appendage; and to derive at least one relation of the at least one first anatomical measurement and the at least one second anatomical measurement; and
wherein the output interface is configured to provide the derived at least one relation as an indicator for a pressure related parameter of the mitral valve.

2. Device according to claim 1, wherein the at least one relation comprises a relative difference between the at least one first anatomical measurement and the at least one second anatomical measurement; and
wherein the derived at least one relation is provided as an indicator for mitral regurgitation.

3. Device according to claim 1 or 2, wherein the first pressure state and the second pressure state relate to one of the group of:
i) a first point and a second point on one pressure curve of a cardiac cycle, wherein the second point is different from the first point; and
ii) a pressure state at a first stage of a cardiac treatment and a pressure state at a second stage of the cardiac treatment, the second stage being different in relation to the proceeding of the cardiac treatment than the first stage; wherein the first pressure state and the second pressure state relate to the same part of a respective cardiac cycle.

4. Device according to claim 3, wherein, for i), the first pressure state relates to a first point on an atrial pressure curve and the second pressure state relates to a second point on the atrial pressure curve;
wherein the first point is a first peak of an a-wave part of the atrial pressure curve and the second point is a second peak of a v-wave part of the atrial pressure curve; and
wherein the at least one relation of the at least one first anatomical measurement and the at least one second anatomical measurement comprises a determination of a relative difference between the first and the second peak in relation to an amplitude of the atrial pressure curve.

5. Device according to claim 3, wherein, for ii), the pressure states at the first and second stage relate to a peak of a v-wave part of the atrial pressure curve;
wherein the at least one relation of the at least one first anatomical measurement and the at least one second anatomical measurement comprises a determination of the peak of the v-wave part of the atrial pressure curve before the treatment and the peak of the v-wave part of the atrial pressure curve after the treatment;
wherein, for the determination, a first geometrical measurement is provided for the peak of the v-wave part of the atrial pressure curve before the treatment, and a second geometrical measurement is provided for the peak of the v-wave part of the atrial pressure curve after the treatment.

6. Device according to one of the preceding claims, wherein the data input is configured to provide an electrocardiogram (24) in order to determine the first point and the second point on the atrial pressure curve.

7. Device according to one of the preceding claims, wherein the first ultrasound image and the second ultrasound image further comprise a mitral valve area of the heart; and
wherein the first and second ultrasound images are:
i) provided by a mitral valve procedure and are used for the determining of the pressure-related value of a mitral valve of a heart; and/or
ii) provided for a mitral valve procedure.

8. Device according to one of the preceding claims, wherein the data processor is configured to provide the segmenting as at least one of the following:
- model-based segmentation;
- deep learning segmentation; and
- manual segmentation based on user input via a user interface.

9. Device according to one of the preceding claims, wherein the data processor is configured to segment a mitral valve image and register the segmentation with a model; and
wherein the data processor is configured to estimate a location of the left atrial appendage in the mitral valve image based on a left atrial appendage location indication of the model.

10. Device according to one of the preceding claims, further comprising a display (26);
wherein the display is configured to display the derived at least one relation.

11. A system (100) for determining a pressure-related value of a mitral valve of a heart, the system comprising:
- an ultrasound imaging device (102) configured to provide image data of the mitral valve of a heart of a subject; and
- a device (10) according to one of the preceding claims;
wherein the ultrasound imaging device provides the first and second ultrasound images of the heart.

12. System according to claim 11, wherein the ultrasound imaging device comprises a transesophageal imaging probe for acquiring the first and second ultrasound images.

13. A method (200) for determining a pressure-related value of a mitral valve of a heart, the method comprising the following steps:
- providing (202) a first ultrasound image of a heart comprising at least a proximal part of a left atrial appendage of the heart in a first state; and
- providing (204) a second ultrasound image of a heart comprising at least the proximal part of the left atrial appendage of the heart in a second state;
wherein the first state relates to a first pressure state of the left atrium of the heart and the second state relates to a second pressure state of the left atrium of the heart which second pressure state is different than the first pressure state;
- segmenting (206) the first ultrasound image generating a first segmentation of at least a part of the left atrial appendage; and segmenting the second ultrasound image generating a second segmentation of at least a part of the left atrial appendage;
- determining (208), from the first segmentation, at least one first anatomical measurement relating to the left atrial appendage; and determining, from the second segmentation, at least one second anatomical measurement relating to the left atrial appendage;
- deriving (210) at least one relation of the at least one first anatomical measurement and the at least one second anatomical measurement; and
- providing (212) the derived at least one relation as an indicator for a pressure related parameter of the mitral valve.

14. A computer program enabling a processor to carry out the method of claim 13.

15. A computer readable medium having stored the program element of claim 14.
